# EUROPEAN PATENT APPLICATION

(11) **EP 4 765 036 A1**
(43) Date of publication of application: **24.06.2026**
(21) Application number: 24220553.2
(22) Date of filing: 17.12.2024
(51) Int. Cl.: G06V 10/764, A61B 5/00, G06V 10/82, G06T 7/00

(54) **TOOTH IDENTIFICATION**

(71) Applicant: Koninklijke Philips N.V., 5656 AE Eindhoven (NL)
(72) Inventor: VLUTTERS, Ruud, Eindhoven (NL); GALLUCCI, Alessio, Eindhoven (NL)
(74) Representative: Philips Intellectual Property & Standards

(57) **Abstract**

Proposed concepts aim to provide schemes, solutions, concepts, designs, methods, and systems pertaining to tooth identification in dental imaging. In particular, embodiments aim to provide accurate and robust methods for classifying or identifying teeth in images from oral scans. Such classification of teeth is proposed to use sequences of images rather than single images, providing more context for prediction. Embodiments may therefore enable reliable tooth identification and classification without requiring a highly accurate universal tooth classification model.

## Description

### FIELD OF THE INVENTION

The present invention relates to the field of oral care, and in particular the field of tooth identification in dental imaging.

### BACKGROUND OF THE INVENTION

Dental imaging and analysis have become increasingly important tools in oral healthcare, allowing for early detection and monitoring of various dental conditions. Traditional methods of dental examination rely heavily on visual inspection by dental professionals, which can be subjective and may miss subtle changes or early signs of oral issues. Advanced imaging technologies, such as intraoral cameras/scanners and fluorescence-based systems, have emerged to provide more detailed and objective information about oral health.

One significant challenge in the field of dental imaging is the accurate identification and localization of individual teeth within captured images or scans. This task is important for proper diagnosis, treatment planning, and monitoring of dental conditions. However, the wide variety of tooth shapes, sizes, and arrangements among individuals makes it difficult to develop a universal system for tooth identification that works reliably across diverse populations.

Furthermore, the dynamic nature of the oral environment, including factors such as tooth movement, tooth colour, relative camera pose and/or position, dental work, changes in teeth condition and/or presence, orthodontics conditions, and changes in oral hygiene, can complicate the consistent identification of teeth across multiple imaging sessions. This poses a particular challenge for longitudinal monitoring of oral health, where it is essential to track changes in specific teeth over time.

### SUMMARY OF THE INVENTION

The invention is defined by the claims.

According to an aspect of the invention, a method for tooth identification is provided. The method comprises: obtaining a sequence of scan images of a subject's teeth, the scan images being captured during performance of an oral scanning routine on the subject, and each scan image comprising an image of a respective different tooth of the subject; processing each of the scan images with an image classification algorithm to obtain a predicted tooth label for each scan image, the predicted tooth label for a scan image identifying the tooth in the image; and identifying a tooth of a selected scan image in the sequence based on: the predicted tooth label for the selected scan image and the predicted tooth label for at least one of the other scan images in the sequence.

This method allows for improved accuracy in tooth identification by leveraging contextual information from adjacent teeth in the scanning sequence, reducing errors that may occur when classifying teeth based on individual images alone.

Proposed concepts thus aim to provide schemes, solutions, concepts, designs, methods, and systems pertaining to tooth localization and identification in dental imaging. In particular, embodiments aim to provide accurate and robust methods for classifying or identifying teeth in images from oral scans. Such classification of teeth is proposed to use sequences of images rather than single images, providing more context for prediction. Embodiments may therefore enable reliable tooth identification and classification without requiring a highly accurate universal tooth classification model.

Proposed embodiments employ an idea of utilizing a sequence of scan images captured during an oral scanning routine, thereby improving upon traditional single-image classification approaches. Such a sequential approach may leverage contextual information from adjacent teeth, enhancing the accuracy and reliability of tooth identification. This may be particularly advantageous for dealing with variations in tooth appearance, dental work, and common orthodontic issues that may pose challenges for traditional tooth classification/identification methods.

Unlike conventional methods, proposed embodiments do not rely solely on individual tooth identification predictions. Instead, a proposed embodiment may identify a tooth in a selected scan image based on both its predicted label and the predicted labels of other scan images in the sequence. This approach allows for the correction of potential misclassifications by considering the logical arrangement of teeth in the mouth.

Embodiments may incorporate advanced techniques for sequence analysis and correction. These may include the use of the Viterbi detection algorithm, which employs probabilities based on the confusion matrix of the image classification algorithm and tooth adjacency information from standardized tooth enumeration systems. Additionally, embodiments may explore the use of artificial intelligence (AI) models that are trained to predict original tooth label sequences from noisy sequences, further enhancing an ability to handle complex cases and improve over time.

A key advantage of the proposed concept(s) is the potential for integration into oral care devices. By combining an image capture device with the tooth identification system, the invention enables real-time tooth identification and analysis during routine oral care or professional dental examinations. This integration opens up new possibilities for personalized oral health monitoring and targeted treatment planning. For instance, by incorporating the proposed concept(s) into oral care devices with integrated imaging capabilities, such as Intra-Oral Scanners, embodiments may enable a comprehensive approach to oral health monitoring and personalized care. The proposed concept(s) may also be integrated into oral care applications (otherwise referred to as 'apps') used for scanning and visualization or results. Such integration may allow for regular, convenient scanning and analysis of teeth, potentially leading to earlier detection of dental issues and more effective preventive care strategies.

Overall, the proposals may address a significant challenge in dental imaging and analysis, offering a practical concept that has the potential to improve and/or personalize oral healthcare outcomes for a wide range of individuals.

For example, embodiments may enable accurate tooth identification and localization without requiring a highly accurate universal tooth classification model (which is very difficult to build), making it more robust to variations in tooth appearance across individuals.

Embodiments may further comprise obtaining a video of the subject's teeth captured during performance of an oral scanning routine on the subject; and selecting video frames of the video, wherein in each selected video frame a respective different tooth of the subject is substantially in the centre of the field-of-view of the video frame. This may enable efficient capture of high-quality images of individual teeth without requiring precise manual positioning of the imaging device, streamlining the scanning process. It may also allows determination as to whether resulting teeth images are in an expected sequence or not. The sequence of scan images may comprise images of neighbouring teeth or oral surfaces of the subject. Using images of neighbouring teeth provides additional context for tooth identification, improving the accuracy of the classification process.

Identifying a tooth of a selected scan image in the sequence may comprise analysing the predicted tooth label for the selected scan image and the predicted tooth label for at least one of the scan image preceding the selected scan image in the sequence; and the scan image immediately after the selected scan image in the sequence. This analysis of adjacent teeth in the sequence further enhances the accuracy of tooth identification by considering the logical arrangement of teeth in the mouth.

Embodiments may comprise determining a sequence based on the predicted tooth label for the selected scan image and the predicted tooth label for at least one of the other scan images in the sequence; determining if the determined sequence represents an illogical sequence in the subject's teeth; and responsive to an illogical sequence being determined, modifying the predicted tooth label for the selected scan image. This process of detecting and correcting illogical sequences may help to eliminate errors in tooth identification, improving the overall reliability of the method.

Identifying a tooth of a selected scan image in the sequence may comprise processing the predicted tooth label for the selected scan image and the predicted tooth label for at least one of the other scan images in the sequence with a Viterbi detection algorithm. The use of the Viterbi detection algorithm may provide a powerful statistical approach to sequence correction, further enhancing the accuracy of tooth identification.

The Viterbi detection algorithm may use an emission matrix based on the confusion matrix of the image classification algorithm and a transition matrix based on the tooth adjacency information from a tooth enumeration system, and optionally wherein the tooth enumeration system comprises the ISO 3950 system. The Viterbi detection algorithm may also use probabilities from population statistics or any database containing teeth sequences. Incorporating these probabilities and standardized tooth enumeration information may allow for more accurate sequence correction, particularly in cases where the initial classification may be ambiguous.

Identifying a tooth of a selected scan image in the sequence may comprise using an AI model trained to predict original tooth label sequences from noisy sequences (e.g. an alternative or companion to the Viterbi detection algorithm). The use of an AI model for sequence correction may allow for adaptive and potentially more accurate corrections, especially as the model is exposed to more data over time. For instance, one may re-train the image classifier to predict the sequence corrected tooth numbers, effectively personalizing the image classifier.

The AI model may comprise a stack of one-dimensional convolutional layers. This specific architecture is particularly well-suited for processing sequential data, allowing for efficient and effective correction of tooth label sequences.

The oral scanning routine may be performed without guidance. Allowing for unguided scanning simplifies the user experience and makes the method more accessible to non-professional users.

Scan images may be acquired by an oral care device having an image capture device adapted to capture images of one or more oral features of the subject. Integrating the image capture capability into an oral care device allows for convenient and regular tooth scanning as part of routine oral care.

According to another aspect of the invention, there is provided a computer program product for tooth identification is provided. The computer program product comprises a computer-readable storage medium having program instructions embodied therewith, the program instructions executable by a processor to cause the processor to perform a method comprising: obtaining a sequence of scan images of a subject's teeth, the scan images being captured during performance of an oral scanning routine on the subject, and each scan image comprising an image of a respective different tooth of the subject; processing each of the scan images with an image classification algorithm to obtain a predicted tooth label for each scan image, the predicted tooth label for a scan image identifying the tooth in the image; and identifying a tooth of a selected scan image in the sequence based on: the predicted tooth label for the selected scan image and the predicted tooth label for at least one of the other scan images in the sequence.

Such a computer program product may enable the implementation of the tooth identification method on various computing devices, allowing for widespread adoption and use of the proposed concept(s).

According to another aspect of the invention, there is provided a system for tooth identification. The system comprises: an interface configured to obtain a sequence of scan images of a subject's teeth, the scan images being captured during performance of an oral scanning routine on the subject, and each scan image comprising an image of a respective different tooth of the subject; a processor arrangement configured to process each of the scan images with an image classification algorithm to obtain a predicted tooth label for each scan image, the predicted tooth label for a scan image identifying the tooth in the image; and a controller configured to generate a signal identifying a tooth of a selected scan image in the sequence based on: the predicted tooth label for the selected scan image and the predicted tooth label for at least one of the other scan images in the sequence.

This system provides a comprehensive hardware and software solution for implementing the tooth identification method, enabling efficient and accurate tooth identification in various dental applications.

The interface may comprise an image capture device adapted to capture images of one or more oral features of the subject. Integrating the image capture device directly into the system allows for seamless acquisition of tooth images, simplifying the overall tooth identification process.

The system may be incorporated into an oral care device comprising an image capture device adapted to capture images of one or more oral features of a user. The oral care device may, for example, comprise an Intra-Oral Scanner. Integrating the tooth identification system into an oral care device with an Intra-Oral Scanner creates a comprehensive solution for dental imaging and analysis, enabling advanced oral health monitoring and personalized care recommendations.

Embodiments may therefore be of particular relevance to oral care devices having an image capture device (e.g. digital camera), such as Intra-Oral Scanners (IOSs), dental/oral cameras and electric toothbrushes for example. Thus, according to another aspect of the invention, there may be provided an oral care device having: an image capture device adapted to capture images of one or more oral features of a user; and a system according to a proposed embodiment, and optionally wherein the oral care device comprises an Intra-Oral Scanner.

Such an integrated oral care device may combine tooth imaging and localization capabilities, potentially enabling real-time tooth identification and analysis during routine oral care or professional dental examinations.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a flowchart of a method for tooth identification using scan images, according to aspects of the present disclosure.
Fig. 2A depicts a screen capture of a classical Viterbi detection algorithm on simulated sequences, in accordance with an embodiment.
Fig. 2B depicts a screen capture of a double Viterbi detection algorithm on simulated sequences, according to an aspect of the present disclosure.
Fig. 3 illustrates a schematic drawing of a model for predicting the class of a middle image, according to an embodiment.
Fig. 4 illustrates a block diagram of a system for tooth identification, according to an example embodiment.
Fig. 5 illustrates a side view of an oral care device integrated with the system of Fig. 4, according to aspects of the present disclosure.
Fig. 6 illustrates a block diagram of a computing system, in accordance with example embodiments.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes and/or solutions pertaining to assessing an oral health care routine of a user. According to proposed concepts, a number of possible solutions may be implemented separately or jointly. That is, although these possible solutions may be described below separately, two or more of these possible solutions may be implemented in one combination or another.

The invention proposes concepts for tooth identification that utilizes a sequence of scan images captured during an oral scanning routine. The concepts improve upon traditional single-image identification/classification approaches by leveraging contextual information from adjacent teeth and/or oral surfaces, enhancing the accuracy and reliability of tooth identification. The proposed concept(s) may also be extended to include the tooth surface (e.g. occlusal/lingual/buccal or differently named chewing/inner/outer, etc.).

Embodiments process multiple scan images with an image classification algorithm to obtain predicted tooth labels, then identifies teeth based on both the predicted label for a selected scan image and the labels of other images in the sequence. This sequential approach may be particularly advantageous for dealing with variations in tooth appearance, dental work, and common orthodontic issues that may pose challenges for traditional tooth classification methods.

The proposed inventions may incorporate advanced techniques for sequence analysis and correction, such as the Viterbi detection algorithm, which employs probabilities based on the confusion matrix of the image classification algorithm and tooth adjacency information from standardized tooth enumeration systems. Additionally, embodiments may use artificial intelligence (AI) models trained to predict original tooth label sequences from noisy sequences, further enhancing the ability to handle complex cases and improve over time.

The proposed concepts offer a robust and efficient solution for tooth identification that do not rely on single scan images. Instead, embodiments capitalize on contextual information from adjacent teeth and/or oral surfaces, allowing for accurate tooth identification even in the face of variations in tooth appearance, dental work, and/or common orthodontic issues. That is, the invention proposes tooth identification that utilizes sequences of scan images rather than relying on single images. This approach significantly improves the accuracy of tooth classification by providing more context for the image classification algorithm. This may be particularly advantageous for longitudinal monitoring of oral health, where it is essential to track changes in specific teeth or oral features/surfaces over time.

A key benefit of the proposed invention is its potential for integration into oral care devices with imaging capabilities, such as intra-oral scanners or electric toothbrushes. Such integration may enable real-time tooth identification and analysis during routine oral care or professional dental examinations, opening up new possibilities for personalized oral health monitoring and targeted treatment planning. For example, embodiments may be implemented in an oral care device equipped with an image capture device, such as an intraoral scanner, thus enabling regular, convenient scanning and analysis of teeth. Such integration may lead to earlier detection of dental issues and more effective preventive care strategies.

Also, an ability to work with unguided scanning routines may also simplify the user experience, making it more accessible to non-professional users. Overall, the proposed invention may address a significant challenge in dental imaging and analysis, offering a practical solution that has the potential to improve oral healthcare outcomes for a wide range of individuals. By enabling accurate tooth identification without requiring a highly accurate universal tooth classification model, the proposed invention may be more robust to variations in tooth appearance across individuals.

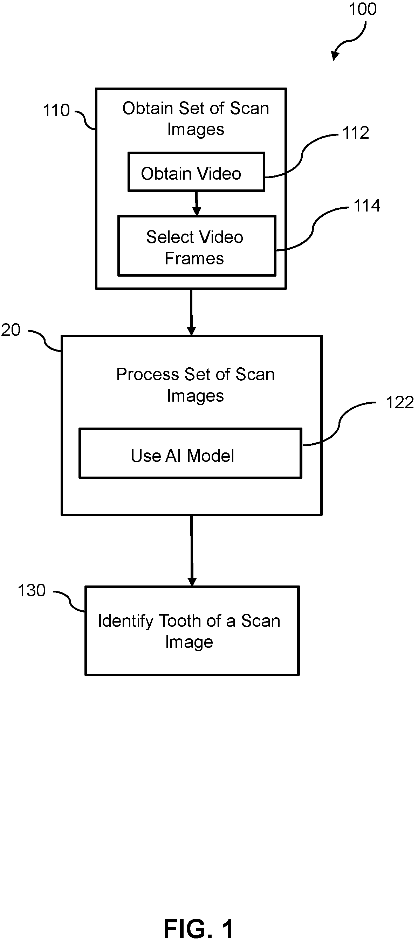

Referring now to Fig. 1, there is depicted a flow diagram of a method 100 for tooth identification according to a proposed embodiment. The flow diagram depicts a sequential process where each step leads to the next, showing how the method 100 progresses from data collection to analysis and tooth identification.

In summary, the method 100 comprises three main steps: obtaining scan images (step 110), processing scan images (step 120), and identifying a tooth (step 130).

In step 110, a sequence of scan images of a subject's teeth is obtained. These scan images are captured during performance of an oral scanning routine on the subject. Each scan image in the sequence comprises an image of a respective different tooth of the subject.

Step 120 involves processing each of the scan images. An image classification algorithm is applied to each scan image to obtain a predicted tooth label. The predicted tooth label for a scan image identifies the tooth depicted in that image.

In step 130, a tooth of a selected scan image in the sequence is identified. This identification is based on the predicted tooth label for the selected scan image and the predicted tooth label for at least one of the other scan images in the sequence. By considering multiple scan images in the sequence, the method 100 may improve the accuracy of tooth identification compared to analyzing each scan image in isolation.

In more detail, the method 100 begins with step 110 of obtaining a sequence of scan images of a subject's teeth. Each scan image in the sequence comprises an image of a respective different tooth of the subject. The scan images are captured during performance of an oral scanning routine on the subject. In this example, the scan images are acquired by an oral care device (such as an intraoral scanner) having an image capture device adapted to capture images of one or more oral features of the subject.

Specifically, the step 110 obtaining a sequence of scan images of a subject's teeth in the example embodiment of Fig. 1 comprises two sub-steps: step 112 of obtaining a video and step 114 of selecting video frames.

In step 112, a video of the subject's teeth is obtained. The video is captured by an oral care device during performance of an oral scanning routine on the subject.

In step 114, specific video frames are selected from the obtained video. In each selected video frame, a respective different tooth of the subject is substantially in the centre of the field-of-view of the video frame. Suitable image/video processing algorithms and techniques to implement this frame selection concept will be easily accessible and implementable to a skilled reader. By way of example, the frame selection process may comprise instance segmentation, YOLO and bounding boxes (a simple selection metric being whether the bounding box is in the center of the frame). This selection process ensures that each scan image in the sequence comprises an image of a respective different tooth of the subject.

The sequence of scan images obtained through steps 112 and 114 includes images of neighboring teeth or oral surfaces of the subject. This provides context for subsequent processing and identification steps.

Upon completion of step 110, the method 100 proceeds to step 120 of processing the scan images. In step 120, each of the scan images is processed with an image classification algorithm to obtain a predicted tooth label for each scan image. The predicted tooth label for a scan image identifies the tooth in the image. Here, step 120 includes a sub-step 122 that utilizes an AI model to process the scan images. The AI model in sub-step 122 may be trained to recognize and classify different teeth based on their visual characteristics.

By way of further example, the image classification algorithm may use an EfficientNet-B0 model as the encoder for feature extraction. This model may provide good performance while being small enough to run on mobile phone processors or edge devices such as microcontrollers in scanning devices. In some examples, self-supervised methods like BERT (Bidirectional Encoder Representations from Transformers) or contrastive predictive coding may be used for training the encoder and sequence processing block. These methods can leverage large amounts of unlabeled data to improve the model's performance.

After obtaining the predicted tooth labels, the method 100 proceeds to step 130 of identifying a tooth of a scan image. Here, the method identifies a tooth of a selected scan image in the sequence based on the predicted tooth label for the selected scan image and the predicted tooth label for at least one of the other scan images in the sequence. This leverages the concept that the sequence of scan images includes images of neighboring teeth or oral surfaces of the subject. This allows the method to analyze the predicted tooth label for the selected scan image in context with the predicted tooth labels of adjacent scan images in the sequence.

To process the sequence of images, various sequence processing methods may be employed. One approach uses convolutional layers for sequence processing. Another approach utilizes LSTM (Long Short-Term Memory) layers, which can effectively handle sequences of varying lengths. A third approach employs transformer layers, similar to those used in natural language processing tasks. An advantage of these approaches is that they can work with variable sequence lengths.

The sequence-based approach significantly improves classification accuracy compared to single image classification. In experiments using a dataset collected from an internal user study, the sequence-based method achieved an accuracy of 82%, compared to a baseline accuracy of 68% for single image classification. This represents a reduction in error rate of more than 50%.

The improved accuracy is particularly noticeable in reducing confusion between neighboring teeth, which is a common issue with single image classification methods. By considering the context provided by the sequence of images, the method can more accurately distinguish between similar-looking teeth based on their position in the dental arch.

This sequence-based tooth classification method provides a robust foundation for various dental health applications, including plaque detection and monitoring of oral health over time. By accurately identifying individual teeth, the method enables more precise reporting and tracking of dental conditions, ultimately contributing to improved oral healthcare outcomes.

In some embodiments, the identification step 130 may comprise correcting illogical tooth number sequences resulting from the image classification algorithm. This processing can significantly improve the accuracy of tooth identification.

For example, the identification step 130 may determine a sequence based on the predicted tooth labels for multiple scan images in the sequence. The step then determine if the determined sequence represents an illogical sequence in the subject's teeth. If an illogical sequence is determined, the identification step 130 modifies the predicted tooth label for one or more scan images in the sequence.

One approach for such 'post-processing' identification can employ a Viterbi detection algorithm. The Viterbi detection algorithm processes the predicted tooth labels for multiple scan images in the sequence. The algorithm uses probabilities based on a confusion matrix of the image classification algorithm and tooth adjacency information from a tooth enumeration system. In some examples, the tooth enumeration system is the ISO 3950 system.

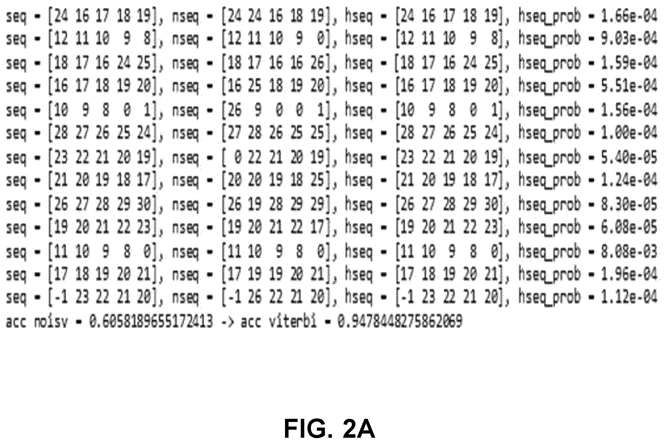

For example, Fig. 2A depicts a screen capture of running the classical Viterbi detection algorithm on simulated sequences. The figure depicts a data table showing results of a tooth classification algorithm. The table contains multiple rows, each representing a different sequence of tooth numbers. The sequence numbers are the ISO 3950 tooth numbers, mapped to [0,1,...,31] labels. A -1 label represents a non-valid tooth number/label. For each row, three sequences are shown: the original sequence (seq), a noisy sequence (nseq), and a hidden sequence (hseq). Additionally, a probability value (hseq_prob) is provided for each hidden sequence. At the bottom of the table, accuracy values are given for both the noisy and Viterbi algorithms. The data illustrates the performance of the tooth classification method across various tooth number sequences.

In another example, the Viterbi algorithm may be applied twice with different transition matrices (i.e. `double Viterbi detection'). A first application uses a transition matrix containing only left-to-right transitions, while a second application uses a transition matrix containing only right-to-left transitions. The method then selects the result with the highest probability as the final solution.

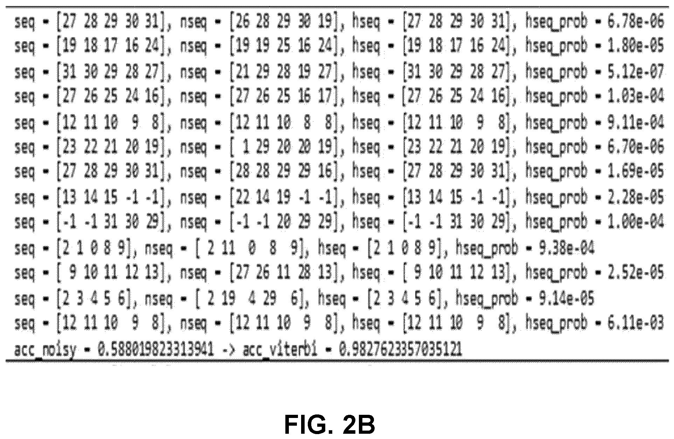

Fig. 2B depicts a screen capture running the double Viterbi detection algorithm on simulated sequences. The sequence numbers are the ISO 3950 tooth numbers, mapped to [0,1,...,31] labels. A -1 label represent a non-valid tooth number/label. The table contains multiple rows, each representing a different sequence of tooth numbers. For each row, there are four columns: the original sequence (seq), a noisy sequence (nseq), a hidden sequence (hseq), and a hidden sequence probability (hseq_prob). At the bottom of the table, two accuracy values are provided: acc_noisy and acc_viterbi, which appear to compare the performance of different classification methods.

Another approach for post-processing identification uses an artificial intelligence (AI) model trained to predict original tooth label sequences from noisy sequences. That is, the Viterbi algorithm may be replaced with a fully convolutional neural network, that can be trained to correct the sequence errors. For example, the AI model can comprise a stack of one-dimensional convolutional layers. The AI model takes as input the noisy labels in one-hot notation and predicts, for each label in the sequence, to which class it belongs in the original sequence.

Both the Viterbi algorithm and AI-based processing approaches have demonstrated substantial improvements in classification accuracy. In experiments, these methods achieved up to 98% accuracy in predicting correct tooth identification numbers.

In some examples, the identification step 130 may use floating point probabilities outputted by the image classification algorithm instead of integer labels. This approach may potentially yield better results as the floating point probabilities may better reflect prediction ambiguities.

The post-processing methods described above for step 130 effectively leverage the context provided by the sequence of scan images to correct errors made by the initial image classification algorithm, resulting in more accurate tooth identification.

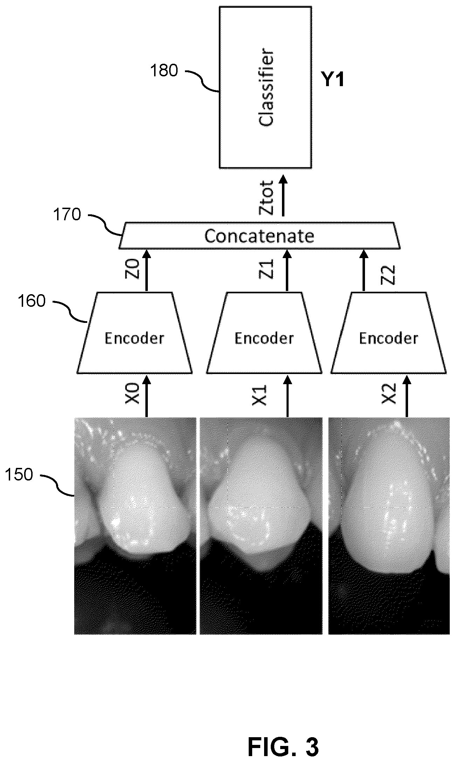

Fig. 3 illustrates a schematic drawing of a tooth identification model according to an exemplary embodiment. The tooth identification model comprises input scan images 150, encoder modules 160, a concatenation module 170, and a classifier module 180.

The tooth identification model receives a sequence of input images 150, labeled X0, X1, and X2. Each of the input images 150 represents a scan image of a subject's teeth captured during an oral scanning routine.

The input images 150 are processed by the encoder modules 160. The encoder modules 160 utilize an EfficientNet-BO model pretrained on the ImageNet dataset. Each of the encoder modules 160 processes one of the input images 150 and generates encoded representations labeled Z0, Z1, and Z2 respectively. The encoder modules 160 output feature maps with 1280 channels for each of the input images 150.

The encoded representations Z0, Z 1, and Z2 are then passed to the concatenation module 170.

The concatenation module 170 combines the encoded representations into a single concatenated representation, labeled Ztot. The concatenated representation Ztot is then input into the classifier module 180.

The classifier module 180 uses a simple linear classifier to process the combined representation and produce an output labeled Y1, which represents the classification result for the middle tooth image.

The tooth identification model may use bi-directional LSTM layers for sequence processing. In some examples, the tooth identification model uses transformer layers for sequence processing. These sequence processing techniques allow the model to consider the context provided by adjacent teeth in the sequence of input images 150.

The tooth identification model may be trained using self-supervised methods such as BERT or contrastive predictive coding for training the encoder modules 160 and sequence processing block. This approach allows the model to leverage large amounts of unlabeled data to improve its performance.

In some examples, the tooth identification model uses an AI model trained to predict original tooth label sequences from noisy sequences. The AI model may comprise a stack of one-dimensional convolutional layers. This configuration allows the model to process the sequence of input images 150 and correct potential misclassifications by considering the overall sequence of teeth.

The tooth identification model as described in Fig. 3 provides a comprehensive approach to processing and classifying teeth images, taking into account the sequential nature of dental scans and the context provided by neighboring teeth.

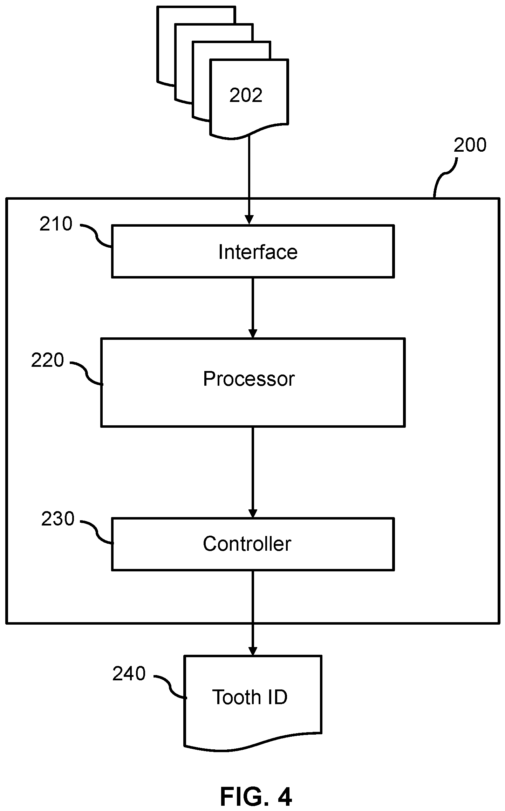

Referring now to Fig. 4, there is depicted a block diagram of a system 200 for tooth identification according to an embodiment of the invention. The system 200 comprises several components that work together to process dental images and identify teeth.

The system 200 includes an input interface 210, a processor 220, and a controller 230. These components are communicatively coupled via a local interface (not shown), which may include additional elements such as controllers, buffers, drivers, repeaters, and receivers to enable communications.

The interface 210 serves as the entry point for image data into the system 200. Here, the interface 210 is configured to obtain a sequence of scan images 202 (e.g. a video) of a subject's teeth. This interface may, for example, be integrated into the oral care device or may be a separate component that communicates with the image capture device. The scan images 202 are captured during performance of an oral scanning routine on the subject.

After obtaining the sequence of scan images 202 (e.g. video), the interface 210 is adapted to select images/frames from the sequence. The selection process focuses on identifying frames where a respective different tooth is substantially in the centre of the field-of-view. This selection process ensures that each scan image in the sequence provides a clear and focused view of a single tooth. The selection of images/frames may involve image processing techniques to identify frames where a tooth is centered. This may include edge detection algorithms to locate tooth boundaries, or centroid calculations to determine the central position of teeth within the frame.

By selecting frames where different teeth are centered, the interface 210 creates a sequence of scan images where each image comprises an image of a respective different tooth of the subject. This approach allows for a comprehensive scan of the subject's dentition, capturing detailed images of each individual tooth.

Once the appropriate video frames are selected, these images/frames become the sequence of scan images used for subsequent processing and tooth identification. This sequence typically includes images of neighboring teeth, providing context for the tooth identification process.

The system 200 further includes a processor 220 connected to the input interface 210. The processor 220 is a hardware device for executing software that can be stored in a memory (not shown). The processor may, for example, include one or more processors, such as a central processing unit (CPU), a graphics processing unit (GPU), or a combination thereof. The processor 220 is configured to process the sequence of scan images with an image classification algorithm to obtain a predicted tooth label for each scan image of the sequence. The predicted tooth label for a scan image 202 identifies the (predicted) tooth depicted in the image.

A controller 230 is connected to the processor 220. The controller 230 manages the overall operation of the system 200, coordinating the functions of the other components and controlling the flow of data and processing. In particular, the controller 230 is configured to generate an output signal 240 identifying a tooth of a selected scan image in the sequence based on: the predicted tooth label for the selected scan image and the predicted tooth label for at least one of the other scan images in the sequence. The identification determined by the controller 230 is based on the predicted tooth label for the selected scan image and the predicted tooth label for at least one of the other scan images in the sequence. By considering multiple scan images, the controller 230 improves accuracy compared to single image classification.

This output signal 240 may be used to indicate the identified tooth in the scan image, providing valuable information about the location and condition of the tooth. The signal 240 may, for example, be a digital signal, an analog signal, or any other type of signal suitable for conveying information about the identified tooth.

Yet further, the controller 230 may be configured to generate a visual representation of the identified tooth based on the generated signal. This visual representation may be displayed on a display device, such as a screen of a smartphone, a tablet, a computer, or any other suitable display device. The visual representation may include a graphical representation of the identified tooth, a textual description of the identified tooth, or any other suitable representation of the identified tooth.

By way of yet further example, the controller 230 may generate additional information about the identified tooth based on the generated signal. This additional information may include information about the condition of the identified tooth, such as the presence of plaque or tartar, the condition of the tooth enamel, or any other relevant information about the tooth. This additional information may be displayed along with the visual representation of the identified tooth, providing the user with comprehensive information about their oral health.

By integrating the components detailed above, the system 200 leverages contextual information from the image sequence to improve tooth identification accuracy. This approach helps overcome limitations of single image classification methods, particularly in distinguishing between similar neighboring teeth.

Variations to the features detailed above for the embodiment of Fig. 2 may be employed.

For example, before proceeding to the classification stage, the selected scan images may undergo pre-processing steps. These pre-processing steps may include image enhancement techniques such as contrast adjustment, noise reduction, or image normalization. These pre-processing steps may improve the quality and consistency of the images, which can enhance the performance of the subsequent image classification algorithm.

By way of further example, in some cases, the input interface 210 may actually comprise/include an image capture device adapted to capture images of oral features of the subject. This image capture device may be part of an oral care device, such as an intra-oral scanner. The image capture device may be adapted to capture images of the subject's teeth under different lighting conditions, such as white light illumination, Quantitative Light-induced Fluorescence (QLF) illumination, or Fluorescence Imaging with Reflectance Enhancement (FIRE) illumination.

In some example implementations, the processor 220 and/or the controller 230 may be configured to perform additional processing tasks, such as image preprocessing, image segmentation, and image classification. The processor 220 and/or the controller 230 may also be configured to perform other tasks related to oral health care, such as plaque detection, tartar detection, and caries detection. These additional tasks may be performed using the same AI model or different AI models, depending on the specific requirements of the tasks.

The processor 220 may be configured to perform the prediction and identification tasks in real-time or near real-time. This may allow for immediate feedback to the user during the oral scanning routine, potentially improving the user's oral care routine and overall oral health. In other cases, the processor 220 may perform these tasks in a batch mode, processing multiple images at once and providing the results at a later time. This may be beneficial in situations where real-time feedback is not necessary or where processing resources are limited.

Some embodiments might recognize multiple teeth instances in the same image and crop them out creating a set of images with one tooth each. Alternatively, some embodiments may employ an image classifier that takes as input an image plus a bounding box, so that it made clear for the image classifier which tooth it has to predict the tooth number for. In this approach, there is no need to crop the image. Such object recognition model can be built with YOLO-v8 or any other opensource alternative such as MMdetection.

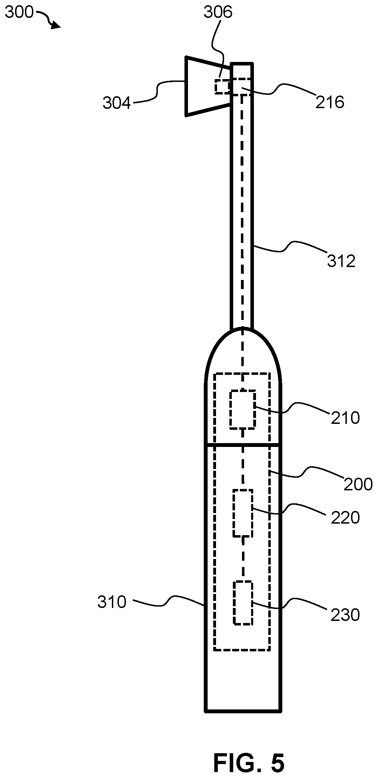

Fig. 5 illustrates a side view of an oral care device 300 incorporating the system 200 of Fig. 4. The oral care device 300 is an intraoral scanner which comprises a handle section 310 atop which is an elongated arm 312.

At the distal end of the arm is a conical section 304 which houses an image capture device 306. The image capture device 306 is adapted to capture high-resolution images of the subject's teeth during the performance of an oral care routine. The Intra-Oral Scanner 300 is thus configured to capture images of one or more oral features of the subject, enabling the acquisition of scan images.

The Intra-Oral Scanner 300 provides several benefits for tooth identification and oral health monitoring. Firstly, it allows for the capture of high-quality images of the subject's teeth, which can enhance the accuracy of the tooth identification process. Secondly, it enables regular, convenient scanning of the subject's teeth during their regular oral care routine, facilitating longitudinal monitoring of oral health. Thirdly, it allows for the capture of images of the subject's teeth in a consistent and standardized manner, which can improve the reliability of the tooth identification process.

In some cases, the Intra-Oral Scanner 300 may be configured to capture images of the subject's teeth under different lighting conditions, such as white light, Quantitative Light-induced Fluorescence (QLF), and Fluorescence Imaging with Reflectance Enhancement (FIRE) imaging mode. This can enhance the visibility of different oral features, such as plaque and tartar, and facilitate their detection and identification.

For example, in a QLF mode, a blue LED light source may be used to illuminate the teeth, and a band pass filter may be placed in front of the camera to block the blue excitation light. This allows the auto-fluorescence of plaque on the teeth to be imaged, providing valuable information about the presence and location of plaque on the teeth. In a FIRE imaging mode, QLF is combined with white light illumination to illuminate the gum (because it does not light up fluorescently), providing a comprehensive view of the teeth and the gum. Such modes can provide valuable information about the presence and location of plaque and tartar on the teeth, aiding in the assessment of the user's oral health.

In some cases, the Intra-Oral Scanner 300 may be configured to capture images of the subject's teeth at different angles and orientations, allowing for comprehensive coverage of each tooth. This can enhance the accuracy of the tooth identification process by providing multiple views of each tooth.

Also provided at the distal end of the arm 312, adjacent the optical sensor 306, is an IMU 216. The IMU 216 is configured to measure the acceleration and angular velocity of the oral care device, providing detailed information about the device's location and/or movement patterns.

The system 200 is integrated within the handle section 310. The input interface 210 of the system is positioned near the top of the handle section 310, to receive data from the image capture device 306. Below the input interface 210 is the processor 220, which is responsible for analysing the data. The controller 230 of the system is situated at the bottom of the handle 300.

Connections link both the image capture device 306 and IMU 216 to the input interface 210, the input interface 210 to the processor 220, and the processor 220 to the controller 230. The connections thus facilitate the flow of data and signals within the system 200.

The arrangement of components within the oral care device 300 allows for efficient data processing and control of the image capture device 306 according to the proposed concept(s). The input interface 210 receives data from the image capture device 306 and IMU 216, the processor 220 analyses this data, and the controller 230 generate appropriate signals based on the result(s) of the analysis.

The oral care device 300 integrates imaging capabilities with motion and position sensing. The scanner head 304 allows for intraoral imaging, while the IMU 216 can detect device motion and orientation. The internal components in the handle section 310 process data and control image capture, enabling automated tooth identification.

Modifications to the aforementioned embodiments may be implemented.

For example, in some embodiments, the oral care device 300 may be configured to provide feedback to the subject during the routine scan. This feedback may include visual indicators, audio signals, or haptic feedback to guide the subject in moving the device over their teeth. The feedback may also include real-time information about the oral health conditions detected in the scan images, such as the presence and location of plaque or tartar on the teeth. This real-time feedback may help the subject to improve their oral care routine and maintain their oral health.

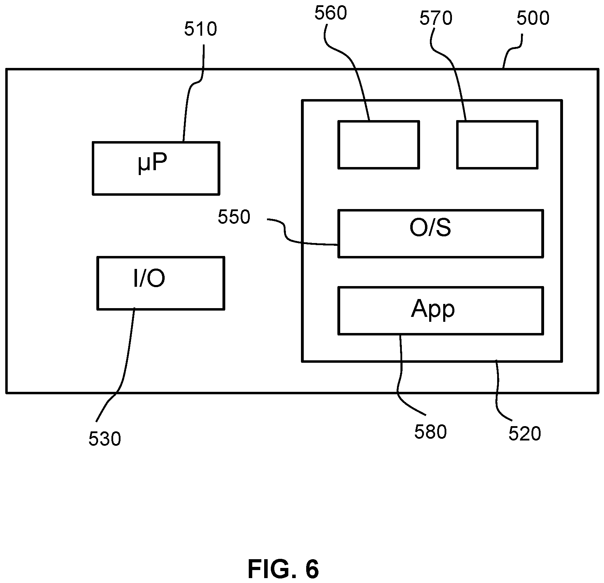

Fig. 6 illustrates an example of a computer 500 within which one or more parts of an embodiment may be employed. Various operations discussed above may utilize the capabilities of the computer 500. In this regard, it is to be understood that system functional blocks can run on a single computer or may be distributed over several computers and locations (e.g. connected via internet).

The computer 500 includes, but is not limited to, PCs, workstations, laptops, PDAs, palm devices, servers, storages, and the like. Generally, in terms of hardware architecture, the computer 500 may include one or more processors 510, memory 520 and one or more I/O devices 530 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor-based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 560, source code 570, and one or more applications 580 in accordance with exemplary embodiments. As illustrated, the application 580 comprises numerous functional components for implementing the features and operations of the exemplary embodiments. The application 580 of the computer 500 may represent various applications, computational units, logic, functional units, processes, operations, virtual entities, and/or modules in accordance with exemplary embodiments, but the application 580 is not meant to be a limitation.

The operating system 550 controls the execution of other computer programs, and provides scheduling, input-output control, file and data management, memory management, and communication control and related services. It is contemplated by the inventors that the application 580 for implementing exemplary embodiments may be applicable on all commercially available operating systems.

Application 580 may be a source program, executable program (object code), script, or any other entity comprising a set of instructions to be performed. When a source program, then the program is usually translated via a compiler (such as the compiler 560), assembler, interpreter, or the like, which may or may not be included within the memory 520, so as to operate properly in connection with the O/S 550. Furthermore, the application 580 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, NET, and the like.

The I/O devices 530 may include input devices such as, for example but not limited to, a mouse, keyboard, scanner, microphone, camera, etc. Furthermore, the I/O devices 530 may also include output devices, for example but not limited to a printer, display, etc. Finally, the I/O devices 530 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 530 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at start-up, start the O/S 550, and support the transfer of data among the hardware devices. The BIOS is stored in some type of read-only-memory, such as ROM, PROM, EPROM, EEPROM or the like, so that the BIOS can be executed when the computer 500 is activated.

When the computer 500 is in operation, the processor 510 is configured to execute software stored within the memory 520, to communicate data to and from the memory 520, and to generally control operations of the computer 500 pursuant to the software. The application 580 and the O/S 550 are read, in whole or in part, by the processor 510, perhaps buffered within the processor 510, and then executed.

When the application 580 is implemented in software it should be noted that the application 580 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 580 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The embodiments of the Figures may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method (100) for tooth identification, the method comprising:
obtaining (110) a sequence of scan images of a subject's teeth, the scan images being captured during performance of an oral scanning routine on the subject, and each scan image comprising an image of a respective different tooth of the subject;
processing (120) each of the scan images with an image classification algorithm to obtain a predicted tooth label for each scan image, the predicted tooth label for a scan image identifying the tooth in the image; and
identifying (130) a tooth of a selected scan image in the sequence based on: the predicted tooth label for the selected scan image and the predicted tooth label for at least one of the other scan images in the sequence.

2. The method of claim 1, wherein obtaining (110) a sequence of scan images of a subject's teeth comprises:
obtaining (112) a video of the subject's teeth captured during performance of an oral scanning routine on the subject; and
selecting (114) video frames of the video, wherein in each selected video frame a respective different tooth of the subject is substantially in the centre of the field-of-view of the video frame.

3. The method of any of claims 1 to 2, wherein the sequence of scan images comprises images of neighbouring teeth or oral surfaces of the subject.

4. The method of claim 3, wherein identifying a tooth of a selected scan image in the sequence comprises:
analysing the predicted tooth label for the selected scan image and the predicted tooth label for at least one of: the scan image preceding the selected scan image in the sequence; and the scan image immediately after the selected scan image in the sequence.

5. The method of any of claims 1 to 3, wherein identifying a tooth of a selected scan image in the sequence comprises:
determining a sequence based on the predicted tooth label for the selected scan image and the predicted tooth label for at least one of the other scan images in the sequence;
determining if the determined sequence represents an illogical sequence in the subject's teeth; and
responsive to an illogical sequence being determined, modifying the predicted tooth label for the selected scan image.

6. The method of claim 5, wherein identifying a tooth of a selected scan image in the sequence comprises:
processing the predicted tooth label for the selected scan image and the predicted tooth label for at least one of the other scan images in the sequence with a Viterbi detection algorithm,
and optionally wherein processing with a Viterbi detection algorithm comprises applying the Viterbi algorithm twice with different transition matrices, a first application using a transition matrix consisting of left-to-right transitions, and a second application using a transition matrix consisting of right-to-left transitions.

7. The method of claim 6, wherein the Viterbi detection algorithm uses probabilities based on a confusion matrix of the image classification algorithm and tooth adjacency information from a tooth enumeration system, and optionally wherein the tooth enumeration system comprises the ISO 3950 system.

8. The method of claim 5, wherein identifying a tooth of a selected scan image in the sequence comprises:
using an artificial intelligence, AI, model trained to predict original tooth label sequences from noisy sequences.

9. The method of claim 8, wherein the AI model comprises a stack of one-dimensional convolutional layers, Long Short-Term Memory layers, or transformer layers.

10. The method of any of claims 1 to 9, wherein scan images are acquired by oral care device having an image capture device adapted to capture images of one or more oral features of the subject.

11. A computer program product for tooth identification, the computer program product comprising a computer-readable storage medium having program instructions embodied therewith, the program instructions executable by a processor to cause the processor to perform a method comprising:
obtaining (110) a sequence of scan images of a subject's teeth, the scan images being captured during performance of an oral scanning routine on the subject, and each scan image comprising an image of a respective different tooth of the subject;
processing (120) each of the scan images with an image classification algorithm to obtain a predicted tooth label for each scan image, the predicted tooth label for a scan image identifying the tooth in the image; and
identifying (130) a tooth of a selected scan image in the sequence based on: the predicted tooth label for the selected scan image and the predicted tooth label for at least one of the other scan images in the sequence.

12. A system (200) for tooth identification, the system comprising:
an interface (210) configured to obtain a sequence of scan images (202) of a subject's teeth, the scan images being captured during performance of an oral scanning routine on the subject, and each scan image comprising an image of a respective different tooth of the subject;
a processor arrangement (220) configured to process each of the scan images with an image classification algorithm to obtain a predicted tooth label for each scan image, the predicted tooth label for a scan image identifying the tooth in the image; and
a controller (230) configured to generate a signal identifying a tooth of a selected scan image in the sequence based on: the predicted tooth label for the selected scan image and the predicted tooth label for at least one of the other scan images in the sequence.

13. The system of claim 12 wherein the interface (210) comprises an image capture device adapted to capture images of one or more oral features of the subject.

14. The system of claim 12 or 13, wherein the interface is adapted to:
obtain a video of the subject's teeth captured during performance of an oral scanning routine on the subject; and select video frames of the video, wherein in each selected video frame a respective different tooth of the subject is substantially in the centre of the field-of-view of the video frame.

15. An oral care device (300) comprising: an image capture device (306) adapted to capture images of one or more oral features of a user; and a system (200) according to any of claims 12 to 14, and optionally wherein the oral care device comprises an Intra-Oral Scanner.
